# EUROPEAN PATENT SPECIFICATION

(11) **EP 0 791 370 B1**
(45) Date of publication and mention of the grant of the patent: **10.12.2003**
(21) Application number: 97301093.7
(22) Date of filing: 20.02.1997
(51) Int. Cl.: A61M 25/06

(54) **Snap-together integral nose and guard arrangement for a safety catheter**
Zusammenschnappbare einstückige Nase- und Schutz-Anordnung für Sicherheitskatheter
Système monobloc nez et garde encliquetable ensemble pour un cathéter de sécurité

(30) Priority: 21.02.1996 US 604402
(43) Date of publication of application: 27.08.1997
(73) Proprietor: ETHICON, INC., Somerville, NJ 08876 (US)
(72) Inventor: Bogert, David L., Plainville, CN 06062 (US)
(74) Representative: Mercer, Christopher Paul

(56) References cited:
- US-A- 3 592 192
- US-A- 4 964 854
- US-A- 5 279 590
- US-A- 5 562 634

## Description

### BACKGROUND OF THE INVENTION

### 1. Field of the Invention

The present invention relates, in general, to intravenous catheter insertion devices, and in more specific particularity, pertains to a catheter needle tip protector safety mechanism which provides fail/safe protection to clinical personnel against the possibility of accidental punctures by used intravenous (IV) needles through the provision of automatic catheter needle tip protecting structure which becomes operative upon withdrawal of the needle from the body of a patient.

Specifically, the invention is directed to the provision of a snap-together nose and guard arrangement for the safety catheter, which provides for a catheter hub releasing mechanism, and whereby the nose and guard arrangement of the catheter insertion device is constituted of an integral or unitarily molded construction, thereby considerably reducing manufacturing costs in comparison with current structures of this type which are ordinarily assembled from a plurality of discrete components.

In greater particularity, there is provided a unitary or single-piece molded nose and guard arrangement incorporating novel hinged components which will enable the nose and the guard to be molded from a single component constituted of a suitable plastic material, and facilitating the nose to be pivoted about an integral hinge into position so as to snap into the guard to thereby provide an operative nose and guard assembly for the catheter insertion device; and also incorporating a further integral hinge for a lever molded therewith to facilitate detachment of a catheter hub which is positioned on the nose.

The utilization of clinical apparatus in which pointed hollow needles or cannulae are employed in order to puncture the skin of a patient, and especially catheters utilizing such needles to effectuate venipunctures, is well known in the medical art and is widely practiced by physicians and clinical personnel for the purpose of injecting fluids and drugs directly into the bloodstream of patients. Additionally, during surgical operations or procedures it may be frequently required that whole blood transfusions and parenteral fluids be administered to a patient undergoing such surgical procedures. Basically, as is well known and has been employed for a considerable length of time, the introduction of such fluids into the cardiovascular systems of patients has necessitated the forming of a venipuncture utilizing a hollow rigid needle having a proximal attachment site for a fluid connection which is adapted to interconnect the needle with a source of intravenously administered fluids.

The foregoing method of administering fluids to patients through venipunctures has been subject to some rather serious problems in the administration of fluids to patients in this medical technology. Thus, a primary concern which had to be addressed resided in the inherent rigidity of the needle, the latter of which is normally generally constituted of surgical-quality steel, and while inserted into the vein of a patient, necessitated the needle to be maintained for reasons of safety in a fixed position at the general site of the venipuncture throughout the duration of fluid administration or transfusion, whereby such a procedure could conceivably consume a considerable length of time. In addition to the foregoing, at times it has been necessary to periodically draw blood samples and/or successively administer intravenous fluids to a patient, thus requiring the patient to be subjected to a series or plurality of venipunctures, each administered at a specific time and at different sites of the body, resulting in a relatively traumatic experience to the patient in view of such repeated and somewhat painful and unpleasant venipunctures.

In order to ameliorate or possibly even eliminate the foregoing problems, in the medical technology it has been more recently the practice to introduce a flexible tubular catheter of a low-friction material, such as a silastic or Teflon into the vein of a patient and to permit the catheter tube to remain in such a position over lengthier periods of time for purposes of; for example, periodically administering fluids, including parenteral fluids, blood/plasma transfusions, medications in liquid form and also for the collection of blood samples and the like. In this manner, the previously encountered trauma, extravasation, and infiltration caused by repeated venipunctures have been largely avoided, and the danger and discomfort to a patient of leaving a rigid needle in the body for a prolonged period of time has been generally overcome. Thus, in order to position the distal end of such a flexible catheter tube within the body cavity of a patient, such as a vascular cavity or vein, there is normally employed a cannula or hollow sharp-tipped needle for the purpose of forming the venipuncture. Thereafter, the flexible catheter tube, which is telescopically and slidably coaxially mounted on the outer circumference of the cannula or hollow needle so as to extend sleeve-like thereabout is advanced along the length of the needle into the vein subsequent to the needle having formed the venipuncture. Thereafter, the needle is adapted to be withdrawn from the interior of the catheter tube, while permitting the latter to remain within the body of the patient at the site of the venipuncture, and the needle is suitably discarded.

Inasmuch as the needle which has been previously positioned in the body of the patient upon forming the venipuncture may have been exposed to infectious agents; for instance, such as a patient infected with the Acquired Immune Deficiency Syndrome (AIDS) which is frequently or practically always ultimately fatal in nature, or other dangerous infectious conditions such as hepatitis, there is present the danger or hazard that the clinical personnel may inadvertently or accidentally jab or stick themselves with the used needle after withdrawal from the body of the patient, with the possibility of infection or even death resulting therefrom.

### 2. Discussion of the Prior Art

Although numerous catheter insertion devices and catheter needle tip protector and safety mechanisms have been developed in this particular technology, generally the nose portion of the catheter insertion device which is adapted to mount the catheter hub is molded as a component which is separate from the needle guard structure which houses the used catheter needle upon withdrawal from the site of the venipuncture implemented on the patient. Although various such devices and arrangements have been developed in the medical art, these are in most instances constituted of a plurality of separate components which are assembled in order to provide an operative assembly, requiring a considerable amount of manual labor to be expended and frequently rendering such devices, which may be of a disposable nature, unduly expensive and possibly uneconomical in use.

Thus, Purdy, et al. U.S. Patent No. 5,215,528 discloses an assembly for introducing a catheter into a blood vessel including a needle hub with a needle secured thereto and which includes a needle guard adapted to have the needle retracted therein. There is, however, no disclosure of an integral nose portion for mounting the needle hub which is integral with the guard of the catheter introducing assembly.

Lituchy U.S. Patent No. 5,205,829 discloses intravenous catheter arrangement in which a needle guard includes a nose member mountable thereon and which provides for the attachment of a catheter hub. In this instance, the nose and guard are formed from two separate molded components; and in essence, require a plurality of parts to provide for the required assembly structure.

Lemieux U.S. Patent No. 5,127,905 discloses a catheter device including a safety needle guard for protecting the needle after use of the device, and wherein a nose portion, detachable from the needle guard, is adapted to mount a hub of a cannula. Again, the structure is constituted of a plurality of separately molded components, rendering the entire arrangement complex and expensive to manufacture.

Luther, et al. U.S. Patent No. 4,950,252 discloses a cannula guard and a housing structure which are mutually relatively axially extendable for receiving therein a used cannula in a protective environment.

McDonald U.S. Patent No. 4,944,725 discloses an intravenous catheter which incorporates a structure for protecting a clinician or physician from accidental puncture which may result in the transfer of dangerous infections from the patient. The catheter is introduced into the patient's body with the aid of a needle of hollow or cannula construction which is thereafter withdrawn from the patient's body into a protective housing in the absence of exposing the needle during any intermediate stage of the withdrawing process. The housing is then latched in place subsequent to needle withdrawal, and for unlocking a catheter hub in place subsequent to that time, and effecting withdrawal and locking in one continuous motion.

Another publication which is applicable in providing for the protection of the point of a needle subsequent to or upon removal thereof from the body of a patient is disclosed in Dombrowski, et al. U.S. Patent No. 4,790,828, wherein a nose portion or cap is tethered to a housing by means of a collapsible tethering structure encompassing the needle such that the needle will be retracted into a sheath-like expanding arrangement which will securely prevent potential injury to clinical personnel caused by being jabbed by an exposed used point of a needle.

US-A-5,279,590, however, discloses an arrangement in which a needle guard means and a catheter hub supporting member are unitarily forming. Its disclosures form the basis for the preamble of claim 1 appended hereto.

### SUMMARY OF THE INVENTION

In essence, although the foregoing needle protecting devices incorporating nose and guard structures are widely utilized, and similar types of arrangements are also known from numerous other U.S. patent publications, none of these provide for a unitarily constructed or molded nose and guard structure incorporating a plurality, preferably.two hinges to enable the deformation or pivoting of the nose relative to the guard in order to form a latching engagement therewith into an operative assembled relationship, and also provide for a cannula hub releasing lever. This type of unitary nose and guard construction for the needle protecting device, although providing a single-piece or integrated molded component, enables in an extremely efficient manner the lowering of manufacturing and assembly costs inasmuch as a plurality of functions are molded into a single plastic element.

Concerning the foregoing, the novel construction of the one-piece or unitarily formed and hingedly connected nose and guard assembly, which incorporates an integral cannula hub releasing lever, assumes a plurality of functions, such as the nose, the guard and the catheter hub lever possessing the capability of releasing a catheter hub mounted on the nose, and which is adapted for a one-handed and user-friendly catheter operation while maintaining the number of molded components of the overall structure at an economical minimum.

Pertaining to the unique unitarily molded construction, the nose is molded in a hinged connection with the leading end of an elongate needle guard element and is adapted to be pivoted about this hinge into assembled alignment therewith and incorporates lever-actuatable latching structure to provide a snap-fitted engagement with the guard in this operative assembled relationship. Furthermore, integrally molded therewith and pivotable about a further or second hinge, so as to provide a so-called "two-hinged" construction, is a further lever which is manually engageable and forwardly deflectable so as to facilitate the pushing-off or dislodging of a catheter hub from the nose, when the catheter hub is mounted thereon in a generally friction-fitting surface engagement.

The integral or unitary construction in one piece of nose, guard and catheter hub release lever and interconnecting hinges enables obtaining a reduction in the cost of manufacturing these components and positioning them into operative interrelationships by considerable factor in comparison with currently employed similar structures incorporating a plurality of separately molded and subsequently piecemeal assembled constituents.

Accordingly, it is an object of the present invention to provide novel unitarily formed or molded nose and guard construction for a needle insertion and protection device.

Another object of the present invention is to provide a unitarily constructed catheter needle protection device incorporating a nose, a needle guard and a catheter hub release lever which are hingedly interconnected and enabling one-handed user-friendly assembly and use thereof.

Still another object of the present invention is to provide a composite hingedly-interconnected and unitary nose, guard and catheter hub release lever for an intravenous catheter insertion device and needle tip protecting structure wherein all of the primary functions thereof are integrated in a single molded and easily manipulated structure.

### BRIEF DESCRIPTION OF THE DRAWINGS

Reference may now be had to the following detailed description of a preferred embodiment of the invention, taken in conjunction with the accompanying drawings; in which:
Figure 1 illustrates, generally diagrammatically, a unitarily molded nose and guard arrangement of a catheter needle tip protective safety mechanism;
Figure 2 illustrates a top plan view of the arrangement of Figure 1;
Figure 3 illustrates a bottom plan view of the arrangement of Figure 1;
Figure 4 illustrates a front end view of the arrangement of Figure 1;
Figure 5 illustrates a side view of the arrangement shown in its snapped-together operative assembly;
Figure 6 illustrates, in more specific detail and on a somewhat enlarged scale, the front end portion of the arrangement of Figure 1;
Figure 7 illustrates a sectional view taken along line 7-7 in Figure 6;
Figure 8 illustrates a sectional view taken along line 8-8 in Figure 6;
Figure 9 illustrates a sectional view taken along line 9-9 in Figure 6;
Figure 10 illustrates a side view of the forward end portion of the arrangement shown in the snapped-together assembled position; and
Figure 11 illustrates a view similar to Figure 10 illustrating a partial longitudinal section thereof.

### DETAILED DESCRIPTION OF A PREFERRED EMBODIMENT

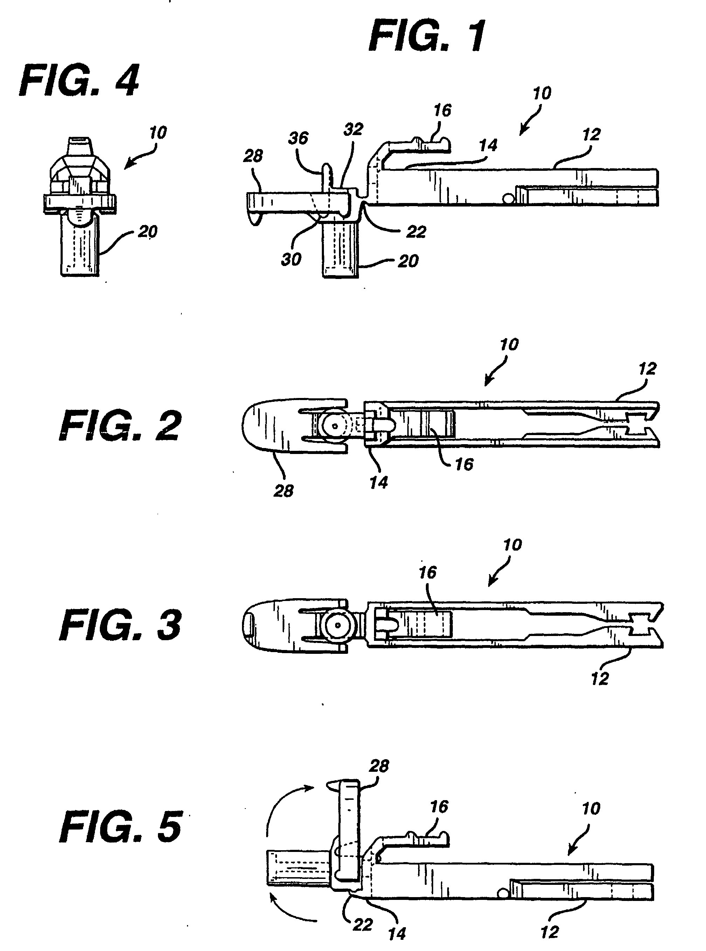

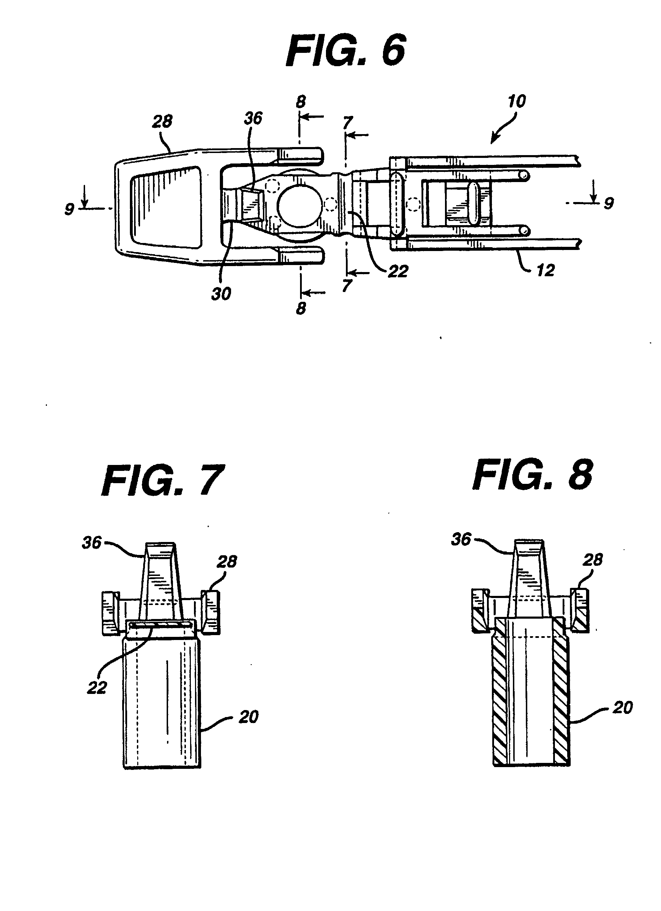

Referring now in specific detail to the drawings, and especially Figures 1 through 8, there is illustrated an arrangement 10 including a guard 12 for a needle utilized in implementing venipunctures or the applications of intravenous fluids, wherein the guard 12 is an elongate member generally box-shaped or U-shaped in transverse cross-section, somewhat similar to that disclosed in EP-A-0 747 087.

The leading end or front end 14 of the guard 12 includes integrally molded therewith an upper rearwardly extending push tab 16 which is essentially a generally flat element, and which is adapted to be engaged in operation of the arrangement by the thumb or a finger of a user. Hingedly connected to and downwardly depending from the front end 14 of the guard 12 at generally a 90° angle is a unitarily therewith molded tubular nose portion 20 connected with the guard through the intermediary of an integrally molded hinge 22 constituted of a thin-walled flexible web connection. The nose portion 20, which is essentially a hollow tubular member, is adapted to have the intravenous needle passed therethrough, and has an outer annular surface adapted to have the hub of a catheter (not shown) mounted thereon, for example, through a friction-contacting engagement therewith. The hub may be seated on the nose portion 20 so that the end wall of the hub (not shown) is located proximate to the hinge portion 22 of the nose 20 adjacent the front end 14 of the guard 12.

Integrally molded with the nose portion 20 and extending at a generally right angle thereto is a lever element 28 for effecting the release of the catheter hub, and whereby the lever element 28 is also essentially pivotable relative to the nose portion through the intermediary of an integrally molded hinge structure 30, also constituted of a thin flexible web connection between the nose portion 20 and lever element 28. The lever element 28 may be an essentially planar plate structure straddling the nose portion 20, as shown in more specific detail in Figures 2 and 3 of the drawings, and which is adapted to be deflected so as to be pivoted relative to the axially assembled position of the nose portion 20 and guard 12, as shown in Figure 5 of the drawings.

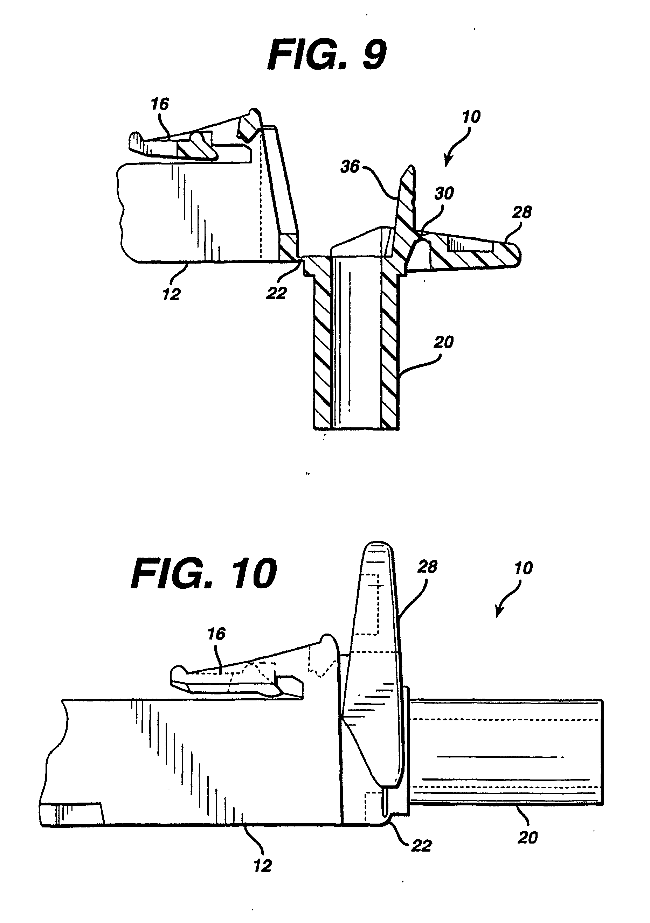

Extending generally coaxially from an end 32 of the nose portion 20 in a direction upwardly at right angles to the longitudinal extent of the guard 12, as shown in Figure 9, is a latch element 36, which, when the nose portion 20 is pivoted about hinge 22 into axial assembled alignment with the guard 12, as shown in Figures 5, 10 and 11 of the drawings, engages by means of recess and detent; in effect, engages into a recess 38 formed in the guard push tab 16, and is latched therein by interengaging projection 40 on the push tab 16 and detent 42 in the latch element 36, so as to cause the nose portion 20 to be fixed in an axial assembled position relative to the elongate guard 12. Release between the nose portion 20 and guard may be effected by merely depressing the push tab 16 so as to disengage the projection 40 from detent 42.

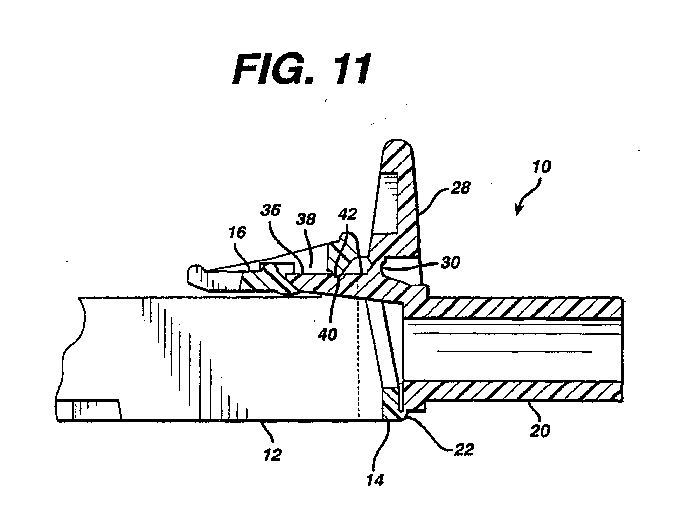

As may be ascertained from Figures 10 and 11 of the drawings, when it is desired to push the catheter or cannula hub (not shown) off the nose portion 20 on which it is seated, subsequent to retracting the needle into the guard; for example, as may be ascertained from EP-A-0 747 087, EP-A-0 747 084 and EP-A-0 747 083 it is merely necessary to apply finger pressure against the catheter hub release lever element 28 in a forward direction so as to pivot the lever element 28 about its hinge 30 connecting it with nose portion 20, thereby causing the hub to slide off or be pushed from the nose portion on which it has been seated.

The foregoing construction is of an extremely simple nature in that it facilitates the entire arrangement 10 consisting of the nose portion 20, guard member 12 and catheter hub releasing lever element 28 to be molded from a single or unitary part in lieu of the heretofore required assembly of these components from a plurality of discrete integers and elements. This facility considerably reduces manufacturing costs and also reduces assembly time for the entire arrangement. Although the economics and savings achieved for each such arrangement in comparison with the multi-component devices of the prior art may be measured only in pennies or small monetary amounts, inasmuch as these arrangements are generally of a single-use or disposable nature and are manufactured and utilized in quantities ranging into the millions, the overall financial savings attained in both manufacturing costs and handling time are quite substantial.

The molding process for achieving the foregoing unitary construction or arrangement 10 is quite simply effectuated, and although appearing to be relatively complex in nature upon a first view, the injection molding of a single plastic constituent which combines a plurality of functions; such as the nose, guard and catheter hub release lever, is accomplished by the use of the integral hinging characteristics imparted to crystalline thermoplastics; for example, such as are selected from the group of materials consisting of polypropylene, polyethylene, nylon, various polyesters, acetals, and so forth.

From the foregoing it becomes readily apparent to one skilled in the art that the construction of the nose, guard and catheter release lever from a single molded constituent considerably reduces manufacturing costs and simplifies handling and assembly thereof while maintaining all of the necessary operative and constructional features provided for in prior art devices of this type.

While there has been shown and described what is considered to be a preferred embodiment of the invention, it will, of course, be understood that it is intended that the invention be not limited to the exact form and detail herein shown and described, nor to anything less than the whole of the invention as hereinafter claimed.

## Claims

1. An arrangement (10) for protecting a cannula of a catheter insertion device, wherein said cannula includes a cannular needle adapted to administer a catheter to a patient and thereafter to be retracted into said catheter insertion device; comprising:
(a) needle guard means (12) for receiving said cannular needle; and
(b) a catheter hub supporting structure including a nose member (20) integrally joined to a leading end of said needle guard means (12), whereby said needle guard means and catheter hub supporting structure are constituted from a unitarily molded structure;
**characterised in that**:
integral hinge means (22) interconnect said needle guard means (12) and said catheter hub supporting structure; and **in that**:
lever means (28) is located on an end of said nose member (20) proximate the hinged connection (22) of said nose member (20) to said needle guard means (12), said lever means (28) being integrally formed with said catheter hub supporting structure; and integral second hinge means (30) join said lever means (28) to said nose member (20).

2. An arrangement (10) as claimed in Claim 1, wherein said integral hinge means (22) comprises a flexible web connecting said nose member (20) to the leading end of said needle guard means (12), said nose member (20) extending at an angular relationship to said needle guard means (12) during the initial forming of said components and being pivotable about said hinge means (22) into a coaxial orientation into the operative assembly of said components.

3. An arrangement (10) as claimed in Claim 1 or 2, wherein said nose member (20) comprises a tubular element, said integral hinge means (22) connecting one end of said tubular element to said needle guard means (12).

4. An arrangement (10) as claimed in Claim 1, 2 or 3, wherein push tab means (16) is formed on the leading end of said needle guard means (12); latch means (36) on said nose member (20) being engageable in a cutout formed in said push tab means (16) in a coaxially assembled orientation of said catheter hub supporting structure and said needle guard means (12).

5. An arrangement (10) as claimed in Claim 4, wherein a projection in said cutout is engageable with a detent on said latch means in the assembled orientation of said components so as to latch said components in the coaxially assembled orientation thereof.

6. An arrangement (10) as claimed in Claim 4 or 5, wherein said latch means (36) comprises an elongate element extending axially from an end of said nose member (20), and being swung into said cutout of said push tab means (16) upon said nose member (20) being pivoted about said hinge means (22) into a coaxial orientation with said needle guard means (12).

7. An arrangement (10) as claimed in any one of Claims 4 to 7, wherein said latch means (36) is integrally formed with said nose member (20).

8. An arrangement (10) as claimed in any one of the preceding Claims, wherein said second hinge means (30) comprises a flexible web facilitating pivotal deflection of said lever means (28) relative to said nose member (20) responsive to manual pressure being applied to said lever means (28).

9. An arrangement (10) as claimed in Claim 8, wherein said lever means (28) comprises a plate element transversely straddling said nose member (20) and extending radially outwardly thereof, said lever means (28) being adapted to contact a catheter hub seated on said nose member (20) and to dislodge said catheter hub from said nose member (20) responsive to axial pressure being manually exerted thereagainst so as to effectuate the release of the catheter hub from said arrangement (10).

10. An arrangement (10) as claimed in any one of the preceding Claims, wherein the constituents of said arrangement (10) comprise a unitarily molded plastic material.

11. An arrangement (10) as claimed in claim 10, wherein said plastic material comprises a crystalline thermoplastic.

12. An arrangement (10) as claimed in Claim 10, wherein said plastic material is selected from the group of materials consisting of polypropylene, polyethylene, nylon, polyesters and acetals.

## Patentansprüche

1. Anordnung (10) zum Schützen einer Kanüle einer Kathetereinführvorrichtung, wobei die Kanüle eine Kanülennadel umfasst, die angepasst ist, um einem Patienten einen Katheter zu legen und danach in die Kathetereinführvorrichtung zurückgezogen zu werden; umfassend:
(a) Nadelschutzmittel (12) zum Aufnehmen der Kanülennadel; und
(b) eine Kathetersitzstützstruktur umfassend ein Ansatzelement (20), das einstückig mit einem vorderen Ende des Nadelschutzmittels (12) verbunden ist, wobei das Nadelschutzmittel und die Kathetersitzstützstruktur aus einer einstückig geformten Struktur gebildet sind;
**dadurch gekennzeichnet, dass**:
einstückige Gelenkmittel (22) das Nadelschutzmittel (12) und die Kathetersitzstützstruktur miteinander verbinden; und dass:
ein Hebelmittel (28) an einem Ende des Ansatzelementes (20) unmittelbar anschließend der gelenkigen Verbindung (22) des Ansatzelementes (20) mit dem Nadelschutzmittel (12) angeordnet ist, das Hebelmittel (28) einstückig mit der Kathetersitzstützstruktur ausgebildet ist; und ein einstückiges zweites Gelenkmittel (30) das Hebelmittel (28) mit dem Ansatzelement (20) verbindet.

2. Anordnung (10) nach Anspruch 1, wobei das einstückige Gelenkmittel (22) einen flexiblen Steg umfasst, der das Ansatzelement (20) mit dem vorderen Ende des Nadelschutzmittels (12) verbindet, das Ansatzelement (20) sich während der anfänglichen Bildung der Bestandteile winkelig zu dem Nadelschutzmittel (12) erstreckt und über das Gelenkmittel (22) in den funktionsfähigen Aufbau der Bestandteile in eine koaxiale Ausrichtung schwenkbar ist.

3. Anordnung nach Anspruch 1 oder 2, wobei das Ansatzelement (20) ein röhrenförmiges Element umfasst, das einstückige Gelenkmittel (22) ein Ende des röhrenförmigen Elementes mit dem Nadelschutzmittel (22) verbindet.

4. Anordnung (10) nach Anspruch 1, 2 oder 3, wobei ein Druckstreifenmittel (16) auf dem vorderen Ende des Nadelschutzmittels (12) ausgebildet ist; Schnappmittel (36) auf dem Ansatzelement (20) in eine in dem Druckstreifenmittel (16) ausgebildete Aussparung in einer koaxial aufgebauten Ausrichtung der Kathetersitzstützstruktur und des Nadelschutzmittels (12) eingreifen können.

5. Anordnung (10) nach Anspruch 4, wobei ein Fortsatz in der Aussparung mit einer Feststelleinrichtung auf dem Schnappmittel in der aufgebauten Ausrichtung der Bestandteile eingreifen kann, so dass die Bestandteile in der koaxial aufgebauten Ausrichtung davon zusammenschnappen.

6. Eine Anordnung (10) nach Anspruch 4 oder 5, wobei das Schnappmittel (36) ein verlängertes Element umfasst, das sich axial von einem Ende des Ansatzelementes (20) erstreckt, und in die Aussparung des Druckstreifenmittels (16) eingeklappt ist, nachdem das Ansatzelement (20) über das Schwenkmittel (22) in eine koaxiale Ausrichtung mit dem Nadelschutzmittel (12) geschwenkt ist.

7. Anordnung (10) nach einem der Ansprüche 4 bis 7, wobei das Druckstreifenmittel (36) einstückig mit dem Ansatzelement (20) ausgebildet ist.

8. Anordnung (10) nach einem der vorangehenden Ansprüche, wobei das zweite Gelenkmittel (30) einen flexiblen Steg umfasst, der das schwenkbare Durchbiegen des Hebelmittels (28) relativ zu dem Ansatzelement (20) in Reaktion auf manuellen Druck, der auf das Hebelmittel (28) ausgeübt wird, erleichtert.

9. Anordnung (10) nach Anspruch 8, wobei das Hebelmittel (28) ein Plattenelement umfasst, das das Ansatzelement (20) quer überspannt und sich radial nach außen davon erstreckt, das Hebelmittel (28) angepasst ist, um einen Kathetersitz, der auf dem Ansatzelement (20) aufsitzt, zu kontaktieren und den Kathetersitz von dem Ansatzelement in Reaktion auf axialen Druck, der manuell dagegen ausgeübt wird, zu entfernen, um die Freisetzung des Kathetersitzes aus der Anordnung (10) zu bewirken.

10. Anordnung (10) nach einem der vorangehenden Ansprüche, wobei die Bestandteile der Anordnung (10) ein einstückig geformtes Kunststoffmaterial umfassen.

11. Anordnung (10) nach Anspruch 10, wobei das Kunststoffmaterial einen kristallinen Thermoplasten umfasst.

12. Anordnung (10) nach Anspruch 10, wobei das Kunststoffmaterial ausgewählt ist aus der Gruppe umfassend Polypropylen, Polyethylen, Nylon, Polyester und Acetale.

## Revendications

1. Dispositif (10) pour protéger une canule d'un dispositif d'insertion de cathéter, dans lequel ladite canule comprend une aiguille cathéter adaptée pour poser un cathéter sur un patient et pour être, par la suite, rentrée dans ledit dispositif d'insertion de cathéter ; comprenant :
(a) un porte-aiguille (12) pour recevoir ladite aiguille cathéter ; et
(b) un embout de cathéter supportant une structure comprenant un système monobloc nez (20) attaché solidairement à une extrémité principale dudit porte-aiguille (12), moyennant quoi ledit porte-aiguille et l'embout de cathéter supportant la structure sont constitués d'une structure moulée unitairement ;
**caractérisé en ce que** :
le mécanisme à charnière intégral (22) relie ledit porte-aiguille (12) et ledit embout de cathéter supportant la structure ; et **en ce que** :
le mécanisme à levier (28) se situe sur une extrémité dudit système monobloc nez (20), proche de la connexion avec charnière (22) dudit système monobloc nez (20) audit porte-aiguille (12), ledit mécanisme à levier (28) étant formé solidairement dudit embout de cathéter supportant la structure ; et le second mécanisme à charnière intégral (30) relie ledit mécanisme à levier (28) audit système monobloc nez (20).

2. Dispositif (10) selon la revendication 1, dans lequel le mécanisme à charnière intégral (22) comprend une bande flexible reliant ledit système monobloc nez (20) à l'extrémité principale dudit porte-aiguille (12), ledit système monobloc nez (20) s'étendant, en angle, vers ledit porte-aiguille (12), pendant la formation initiale desdits composants, et étant pivotable au niveau dudit mécanisme à charnière (22), dans une orientation coaxiale, au cours de l'assemblage fonctionnel desdits composants.

3. Dispositif (10) selon la revendication 1 ou 2, dans lequel ledit système monobloc nez (20) comprend un élément tubulaire, ledit mécanisme à charnière intégral (22) reliant une extrémité dudit élément tubulaire audit porte-aiguille (12).

4. Dispositif (10) selon la revendication 1, 2 ou 3, dans lequel le mécanisme poussoir (16) est formé sur l'extrémité principale dudit porte-aiguille (12) ; le mécanisme de verrouillage (36) sur ledit système monobloc nez (20) pouvant être engagé dans une entaille formée dans ledit mécanisme poussoir (16), dans une orientation d'assemblage coaxial de ladite structure supportant l'embout de cathéter et dudit porte-aiguille (12) .

5. Dispositif (10) selon la revendication 4, dans lequel une projection dans ladite entaille peut être engagée avec un encliquetage sur ledit mécanisme de verrouillage, dans l'orientation d'assemblage desdits composants, de façon à verrouiller lesdits composants sur leur orientation d'assemblage coaxial.

6. Dispositif (10) selon la revendication 4 ou 5, dans lequel ledit mécanisme de verrouillage (36) comprend un élément allongé s'étendant axialement d'une extrémité dudit système monobloc nez (20), et étant glissé dans ladite entaille dudit mécanisme poussoir (16), sur le ledit système monobloc nez (20) étant pivoté au niveau dudit mécanisme à charnière (22) dans une orientation coaxiale avec ledit porte-aiguille (12) .

7. Dispositif (10) selon l'une quelconque des revendications 4 à 7, dans lequel ledit mécanisme de verrouillage (36) est formé solidairement dudit système monobloc nez (20).

8. Dispositif (10) selon l'une quelconque des revendications précédentes, dans lequel ledit second mécanisme à charnière (30) comprend une bande flexible facilitant la déviation centrale dudit mécanisme à levier (28) par rapport audit système monobloc nez (20) sensible à la pression manuelle appliquée sur ledit mécanisme à levier (28).

9. Dispositif (10) selon la revendication 8, dans lequel ledit mécanisme à levier (28) comprend un élément plat chevauchant transversalement ledit système monobloc nez (20) et s'étendant radialement vers l'extérieur de celui-ci, ledit mécanisme à levier (28) étant adapté pour venir au contact d'un embout de cathéter situé sur ledit monobloc nez (20) et pour déloger ledit embout de cathéter dudit monobloc nez (20), sensible à la pression axiale exercée manuellement à son encontre pour retirer l'embout de cathéter dudit dispositif (10).

10. Dispositif (10) selon l'une quelconque des revendications précédentes, dans lequel les constituants dudit dispositif (10) comprennent une matière plastique moulée unitairement.

11. Dispositif (10) selon la revendication 10, dans lequel ladite matière plastique comprend un thermoplastique cristallin.

12. Dispositif (10) selon la revendication 10, dans lequel ladite matière plastique est choisie dans un groupe de matières comprenant du polypropylène, du polyéthylène, du nylon, des polyesters et des acétals.
